# EUROPÄISCHE PATENTSCHRIFT

(11) **EP 2 768 549 B1**
(45) Veröffentlichungstag und Bekanntmachung des Hinweises auf die Patenterteilung: **26.08.2015**
(21) Anmeldenummer: 12783515.5
(22) Anmeldetag: 18.10.2012
(51) Int. Cl.: A61M 1/34, A61M 1/36

(54) **VERFAHREN SOWIE VORRICHTUNGEN ZUM RETROGRADEN BEFÜLLEN WENIGSTENS EINER CALCIUMLEITUNG EINES EXTRAKORPORALEN BLUTKREISLAUFS**
METHOD AND DEVICES FOR RETROGRADE FILLING OF AT LEAST ONE CALCIUM LINE IN AN EXTRACORPOREAL BLOOD CIRCUIT
PROCÉDÉ ET DISPOSITIFS DE REMPLISSAGE RÉTROGRADE D'AU MOINS UNE LIGNE DE CALCIUM D'UN CIRCUIT SANGUIN EXTRACORPOREL

(30) Priorität: 19.10.2011 DE 102011116262; 19.10.2011 US 201161548745 P
(43) Veröffentlichungstag der Anmeldung: 27.08.2014
(73) Patentinhaber: Fresenius Medical Care Deutschland GmbH, 61352 Bad Homburg (DE); Beden, Josef, 55252 Mainz-Kastel (DE); Kessel-deynet, Olaf, 97299 Zell (DE); Klewinghaus, Juergen, 61440 Oberursel (DE); Pusinelli, Thomas, 63674 Altenstadt (DE); Werner, Pascal, 97532 Üchtelhausen (DE)
(72) Erfinder: BEDEN, Josef, 55252 Mainz-Kastel (DE); KESSEL-DEYNET, Olaf, 97299 Zell (DE); KLEWINGHAUS, Juergen, 61440 Oberursel (DE); PUSINELLI, Thomas, 63674 Altenstadt (DE); WERNER, Pascal, 97532 Üchtelhausen (DE)
(74) Vertreter: Bobbert & Partner Patentanwälte PartmbB
(86) Internationale Anmeldenummer: PCT/EP2012/004351
(87) Internationale Veröffentlichungsnummer: WO 2013/056830

(56) Entgegenhaltungen:
- WO-A1-99/37335
- WO-A1-2009/044221
- DE-A1-102006 039 675
- US-A1- 2004 195 178
- US-A1- 2008 015 487

## Beschreibung

Die vorliegende Erfindung betrifft ein Verfahren zum retrograden Befüllen einer von einer Rückgabeleitung eines extrakorporalen Blutkreislaufs abgehenden Leitung zum Zugeben einer medizinischen Lösung, d.h. in der Medizin verwendeten Lösung, beispielsweise einer Calciumleitung. Des Weiteren betrifft die vorliegende Erfindung eine Steuereinrichtung gemäß Anspruch 9 sowie eine Blut- oder Plasmabehandlungsvorrichtung mit wenigstens einer erfindungsgemäßen Steuereinrichtung gemäß Anspruch 10. Zudem betrifft die vorliegende Erfindung ein digitales Speichermedium gemäß Anspruch 12 und ein Computerprogramm-Produkt gemäß Anspruch 13.

Aus der Praxis sind extrakorporale Blutkreisläufe zum Führen von Blut außerhalb des Patientenkörpers während eines Blutreinigungsverfahrens bekannt. Beispiele hierfür werden in den Anmeldungen US 2008/015487 A1, US 2004/195178 A1, WO 2009/044221 A1 und WO 99/37335 A1 beschrieben. Derartige Blutkreisläufe werden vor Beginn der Behandlung mit einer Priminglösung befüllt. Dieses Befüllen ist als Primen des Blutkreislaufs bekannt. Es dient u. a. dazu, im Blutkreislauf vor der Behandlung noch vorhandene Luft durch eine physiologisch verträgliche Flüssigkeit oder Lösung zu verdrängen, um ein Einbringen dieser Luft in das Gefäßsystem des Patienten während und insbesondere zu Beginn der Blutbehandlung zu verhindern.

Weist der extrakorporale Blutkreislauf eine Leitung zum Zugeben von einer medizinischen Lösung, beispielsweise einer Calcium aufweisenden Lösung (hier allgemein als Leitung zum Zugeben einer medizinischen Lösung oder als Calciumleitung, d. h. eine Leitung, welche zum Zugeben oder zum ausschließlichen Zugeben von Calcium oder Calciumlösung in einen extrakorporalen Blutkreislauf vorgesehen ist, bezeichnet) auf, so muss auch diese vor Behandlungsbeginn, jedenfalls aber vor deren tatsächlicher Nutzung, geprimt werden.

Eine Aufgabe der vorliegenden Erfindung ist es, ein Verfahren zum Primen einer Leitung zum Zugeben einer medizinischen Lösung, insbesondere einer Calciumleitung, vorzuschlagen. Die erfindungsgemäße Aufgabe wird durch ein Verfahren mit den Merkmalen des Anspruchs 1 gelöst. Sie wird ferner durch eine Steuereinrichtung mit den Merkmalen des Anspruchs 9 sowie durch eine Blut- oder Plasmabehandlungsvorrichtung mit den Merkmalen des Anspruchs 10 gelöst. Die erfindungsgemäße Aufgabe wird ferner gelöst durch ein digitales Speichermedium, und ein Computerprogramm-Produkt gemäß den Ansprüchen 12 und 13. Alle mit dem erfindungsgemäßen Verfahren erzielbaren Vorteile lassen sich in bestimmten erfindungsgemäßen Ausführungsformen ungeschmälert auch mit den erfindungsgemäßen Vorrichtungen erzielen.

Erfindungsgemäß wird somit ein Verfahren zum Primen und/oder Befüllen und/oder Spülen einer Leitung zum Zugeben einer medizinischen Lösung, und insbesondere einer Calciumleitung, vorgeschlagen, welche von einer Rückgabeleitung eines extrakorporalen Blutkreislaufs abgeht, mit dieser an einem Verbindungspunkt in Fluidkommunikation verbunden ist oder in diese mündet. Dabei werden im Folgenden die Begriffe "Leitung zum Zugeben einer medizinischen Lösung" und "Calciumleitung" nebeneinander verwendet, wobei der Einfachheit halber auch nur von einer Calciumleitung die Rede ist, selbst wenn das hierzu Ausgeführte für den Fachmann erkennbar auch für eine Leitung zutrifft, welche nicht für die Zugabe von Calcium vorgesehen oder verwendet wird. Es wird hiermit klar gestellt, dass stets dann, wenn von einer Calciumleitung die Rede ist, dieser Begriff auch durch den allgemeineren Begriff "Leitung zum Zugeben einer medizinischen Lösung" ersetzbar ist. Die Verwendung des Begriffs Calciumleitung soll die vorliegende Erfindung nicht hierauf beschränken. Eine Calciumleitung ist vielmehr nur eine Ausführungsform der erfindungsgemäßen Leitung zum Zugeben einer medizinischen Lösung, beispielsweise einer Medikamentenlösung, einer Volumensubstitutionslösung, usw. Der Begriff Lösung ist dabei in manchen erfindungsgemäßen Ausführungsformen als ein beliebiges Fluid zu verstehen, welches feste oder gelöste Bestandteile aufweist, also auch Suspensionen, Emulsionen, und dergleichen. Ebenso sind die hierin als Calciumslösungsbehälter, Calciumlösungsanschluss, Calcium-Tropfkammer, Calciumpumpe und dergleichen bezeichneten Gegenstände nicht auf ihre Verwendung im Zusammenhang mit Calcium oder Calciumlösung beschränkt. Auch hier ist "Calcium-" austauschbar gegen jede geeignete medizinische Lösung. Dies liegt ebenfalls im Umfang der vorliegenden Erfindung.

Das Verfahren umfasst ein retrogrades Primen der Calciumleitung über die Rückgabeleitung des extrakorporalen Blutkreislaufs mit einer Priminglösung. Dabei erfolgt das retrograde Primen während des Primens des extrakorporalen Blutkreislaufs, welches mittels einer Blut- oder Plasmabehandlungsvorrichtung wie z. B. einer Apheresevorrichtung, Hämodialyse, Hämofiltration, Hämodiafiltration, usw. erfolgt.

Die erfindungsgemäße Steuereinrichtung ist vorgesehen, eingerichtet, programmiert und/oder konfiguriert zum Steuern oder Regeln einer Vorrichtung zum oder beim Durchführen des erfindungsgemäßen Verfahrens.

Die erfindungsgemäße Blut- oder Plasmabehandlungsvorrichtung weist wenigstens eine erfindungsgemäße Steuereinrichtung auf und/oder ist vorgesehen und/oder eingerichtet zum Ausführen des erfindungsgemäßen Verfahrens. Hierzu erforderliche Einrichtungen weist sie in bestimmten Ausführungsformen auf oder ist mit solchen jeweils in Wirk- und/oder Signalverbindung verbunden.

Das erfindungsgemäße digitale Speichermedium, insbesondere in Form einer Diskette, CD oder DVD, mit elektronisch auslesbaren Steuersignalen kann derart mit einem programmierbaren Computersystem zusammenwirken, dass die maschinellen Schritte des erfindungsgemäßen Verfahrens veranlasst werden.

Das erfindungsgemäße Computerprogramm-Produkt weist einen auf einem maschinenlesbaren Träger gespeicherten Programmcode zur Veranlassung der maschinellen Schritte des erfindungsgemäßen Verfahrens, wenn das Computerprogramm-Produkt auf einem Rechner abläuft, auf.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte und dergleichen sein.

Ein nicht beanspruchtes Computerprogramm weist einen Programmcode auf zur Veranlassung der maschinellen Schritte des erfindungsgemäßen Verfahrens, wenn das Computerprogramm auf einem Computer abläuft.

Für das digitale Speichermedium, das erfindungsgemäße Computerprogramm-Produkt und das Computerprogramm gilt, dass alle, einige oder manche der maschinell durchgeführten Schritte des erfindungsgemäßen Verfahrens veranlasst werden.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern. Vorteilhafte Weiterentwicklungen der vorliegenden Erfindung sind jeweils Gegenstand von Unteransprüchen und Ausführungsformen.

Erfindungsgemäße Ausführungsformen können eines oder mehrere der im Folgenden genannten Merkmale aufweisen.

In bestimmten erfindungsgemäßen Ausführungsformen des Verfahrens ist die Rückgabeleitung vorgesehen zum Rückführen einer extrakorporal behandelten Körperflüssigkeit in das Gefäßsystem des behandelten Patienten. In manchen erfindungsgemäßen Ausführungsformen ist die Rückgabeleitung die venöse Patientenleitung.

Der Blutkreislauf dient in manchen erfindungsgemäßen Ausführungsformen dem extrakorporalen Führen von Blut wie Vollblut oder Bestandteilen hiervon, aber auch von anderen Körperfluiden, etwa Plasma.

In einigen erfindungsgemäßen Ausführungsformen ist "retrograd" jene Strömungsrichtung innerhalb der Calciumleitung, in welcher ein Fluid entgegengesetzt zu jener Strömungsrichtung (der anterograden Strömungsrichtung) strömt, in welcher Calcium durch die Calciumleitung strömt, wenn es während oder gegen Ende der Behandlung des Patienten aus einer Calciumquelle wie einem Calciumlösungsbehälter oder Calciumbeutel heraus und durch die Calciumleitung hindurch in den extrakorporalen Blutkreislauf eingeführt wird.

"Retrogrades Primen oder Befüllen einer Calciumleitung" bedeutet in einigen erfindungsgemäßen Ausführungsformen ein Befüllen der Calciumleitung in einer Richtung zu einem Calciumlösungsbehälter hin (und eben nicht von diesem weg), und/oder zu einer Stelle hin, an welcher der Calciumlösungsbehälter bei seinem üblichem oder bestimmungsgemäßen Gebrauch oder bei Benutzung der Calciumleitung mit Bezug auf diese vorgesehen ist oder wäre.

In gewissen erfindungsgemäßen Ausführungsformen erfolgt das retrograde Primen über einen lösbaren oder unlösbaren Verbindungsabschnitt, mittels welchem die Calciumleitung mit der Rückgabeleitung verbunden ist oder wird.

In bestimmten erfindungsgemäßen Ausführungsformen ist die Priminglösung eine beliebige zum Primen eines extrakorporalen Blutkreislaufs verwendete oder hierfür üblicherweise verwendete oder bekanntermaßen verwendbare Flüssigkeit, beispielsweise physiologische NaCl-Lösung, Dialysierflüssigkeit, Substituat oder dergleichen.

In manchen Ausführungsformen des erfindungsgemäßen Verfahrens ist die Calciumleitung mit der Rückgabeleitung des extrakorporalen Blutkreislaufs einer Blut- oder Plasmabehandlungsvorrichtung mittels eines Verbindungsabschnittes, etwa eines T-Stücks, lösbar oder unlösbar in Fluidkommunikation verbunden.

In einigen erfindungsgemäßen Ausführungsformen umfasst das Verfahren das Nichteinlegen, das Lösen oder Herausnehmen oder das Trennen eines Pumpschlauchabschnittes der Calciumleitung aus oder von einer Calciumpumpe (im Folgenden auch als "Lösen" zusammengefasst).

Das Nichteinlegen oder das Lösen erfolgen in gewissen erfindungsgemäßen Ausführungsformen vor dem Beginn des Primens des extrakorporalen Blutkreislaufs und/oder vor Abschluss des Primens des extrakorporalen Blutkreislaufs.

In bestimmten erfindungsgemäßen Ausführungsformen umfasst das Verfahren das Unterbrechen, Modifizieren (im Sinne eines Veränderns oder Abwandelns) oder Beenden des retrograden Primens der Calciumleitung wenn, falls, sobald oder nachdem ein vorbestimmter Detektionsspiegel der Priminglösung in einer Calcium-Tropfkammer, welche in oder an der Calciumleitung angeordnet ist, erreicht und/oder festgestellt ist.

Das erfindungsgemäße Verfahren umfasst in manchen Ausführungsformen das Unterbrechen oder Beenden des retrograden Primens der Calciumleitung, wenn, falls, sobald oder nachdem ein vorbestimmter Detektionsspiegel der Priminglösung in der Calcium-Tropfkammer erreicht oder und/oder festgestellt ist.

Das Erreichen des vorbestimmten Detektionsspiegels wird in einigen erfindungsgemäßen Ausführungsformen von einer Calcium-Tropf- und Spiegeldetektoreinrichtung erkannt, welche an der Tropfkammer angeordnet ist.

In einigen erfindungsgemäßen Ausführungsformen wird das Erreichen des vorbestimmten Detektionsspiegels automatisch oder automatisiert erkannt.

In gewissen erfindungsgemäßen Ausführungsformen umfasst das Verfahren zusätzlich das weitere Befüllen der Calcium-Tropfkammer mit der Priminglösung, und zwar um ein zusätzliches, vorbestimmtes Volumen der Priminglösung, nachdem der vorbestimmte Detektionsspiegel erreicht und/oder festgestellt ist. Das Volumen dieser Priminglösung kann beispielsweise 3 ml betragen. Das Volumen kann hiervon allerdings abweichen, und es kann von Art und Typ der verwendeten Bauteile oder Komponenten abhängen. Auf diese Weise wird ein geeigneter oder gewünschter After-Priming-Spiegel der Priminglösung in der Calcium-Tropfkammer auch über jenen Zeitpunkt hinaus sichergestellt, an welchem der Pumpschlauchabschnitt in die Calciumpumpe eingelegt wird. Ein solches Einlegen bewirkt ein Absinken des Flüssigkeitsspiegels in der Calcium-Tropfkammer. Dies kann auf die hier beschriebene Weise ganz oder teilweise kompensiert oder überkompensiert werden.

In einigen erfindungsgemäßen Ausführungsformen umfasst das Verfahren das Feststellen eines vorbestimmten Detektionsspiegels in der Calcium-Tropfkammer.

Das erfindungsgemäße Verfahren umfasst in bestimmten Ausführungsformen zusätzlich das Einlegen des Pumpschlauchabschnittes in die Calciumpumpe.

In manchen erfindungsgemäßen Ausführungsformen erfolgt das Einlegen des Pumpschlauchabschnitts in die Calciumpumpe automatisch. Entsprechende Einrichtungen, vorzugsweise auch Mittel zu deren automatischer Ingangsetzung, können erfindungsgemäß an der Blut- oder Plasmabehandlungsvorrichtung vorgesehen oder hiermit verbunden sein.

In bestimmten erfindungsgemäßen Ausführungsformen erfolgt das Einlegen des Pumpschlauchabschnitts in die Calciumpumpe, nachdem der vorbestimmte Detektionsspiegel der Priminglösung in der Calcium-Tropfkammer erreicht und/oder festgestellt wurde.

Das erfindungsgemäße Verfahren umfasst in einigen Ausführungsformen zusätzlich das Verändern des Durchflusses - oder der Strömungsrate der Priminglösung durch die Rückgabeleitung - mittels einer Drosseleinrichtung, einer Stromunterbrechungseinrichtung oder einer Klemmeinrichtung (welche hierin zur Vereinfachung kurz als Klemmeinrichtungen bezeichnet werden, ohne hiermit eine Beschränkung auf eine Einrichtung mit einer expliziten Klemmwirkung vorzunehmen), welche an der Rückgabeleitung stromabwärts des Verbindungsabschnittes oder stromabwärts eines Verbindungspunkts zur Calciumleitung angeordnet ist.

Falls der vorbestimmte Detektionsspiegel - oder der After-Priming-Spiegel - in der Calcium-Tropfkammer nicht während des regulären Primens erreicht wird, so wird in gewissen erfindungsgemäßen Ausführungsformen mittels der Klemmeinrichtung ein Flüssigkeitsdurchfluss innerhalb der Rückgabeleitung stromabwärts des Verbindungspunktes zwischen Rückgabeleitung und Calciumleitung manuell oder automatisch gedrosselt oder unterbunden. Dies erfolgt z. B. indem die Klemmeinrichtung eingerastet wird. Eine entsprechende Aufforderung kann an den Benutzer ergehen, etwa akustisch, optisch, per Displayanzeige, usw. Ein Drosseln, Begrenzen oder Unterbinden kann alternativ automatisch erfolgen, beispielsweise nach Erhalt eines geeigneten Sensorsignals durch einen vorgesehenen Sensor. Ein derartiges Drosseln, Klemmen oder dergleichen kann selbstverständlich auch standardmäßig und - unabhängig hiervon - auch ohne Aufforderung erfolgen.

In bestimmten Ausführungsformen weist die erfindungsgemäße Blut- oder Plasmabehandlungsvorrichtung wenigstens eine Calcium-Tropf- und Spiegeldetektoreinrichtung auf. Diese kann zum Detektieren eines Flüssigkeitsspiegels in der Tropfkammer der Calciumleitung des extrakorporalen Blutkreislaufs ausgestaltet sein.

In gewissen erfindungsgemäßen Ausführungsformen weist die Blut- oder Plasmabehandlungsvorrichtung eine Einrichtung zum Einlegen des Pumpschlauchabschnittes in die Calciumpumpe auf. Die erfindungsgemäße Blut- oder Plasmabehandlungsvorrichtung ist in manchen Ausführungsformen als Apheresevorrichtung, Hämodialysevorrichtung, Hämodiafiltrationsvorrichtung, Hämofiltrationsvorrichtung oder einer Kombination hiervon ausgestaltet.

Manche oder alle erfindungsgemäßen Ausführungsformen können einen, mehrere oder alle der oben und/oder im Folgenden genannten Vorteile aufweisen.

Aus der Praxis ist bekannt, dass bei Plasma- oder Blutbehandlungen regelmäßig eine Substitution von Calcium erforderlich sein kann, etwa bei erfolgter Antikoagulation mit Citrat. Ob eine derartige Substitution tatsächlich erforderlich sein wird, kann im Vorfeld der Behandlung nicht stets zuverlässig abgeschätzt werden. Daher ist es wünschenswert, etwa einen Calciumlösungsbehälter (etwa ein Beutel mit Calciumlösung) nur im Bedarfsfall zu öffnen oder anzubrechen, und diesen andernfalls möglichst ungeöffnet zu belassen, um ihn, sollte eine Calcium-Substitution nicht erforderlich sein, in einer später erfolgenden Blut- oder Plasmabehandlung verwenden zu können. Dies gilt insbesondere vor dem Hintergrund, dass das Vorhalten und Lagern eines derartigen Calciumlösungsbehälters ebenso wie sein Entsorgen mit Mühe und Kosten verbunden sind. Letzteres gilt insbesondere für die Anschaffung eines solchen Behälters; sein Inhalt ist teuer.

Sollte der Calciumlösungsbehälter hingegen bei der Behandlung verwendet werden müssen, etwa aufgrund einer entstehenden Hypokalzämie oder einer erkannten Citratunverträglichkeit, so muss die Calciumleitung vor Einleiten von Calciumlösung zuverlässig geprimt und somit zum Vermeiden von Embolien von Luft entleert sein.

Calciumlösungsbehälter werden mittels der Calciumleitung üblicherweise unmittelbar am Rückgabeanschluss des extrakorporalen Blutkreislaufs mit diesem verbunden, beispielsweise um eine Filterung des eingeleiteten Calciums zu vermeiden, ferner da sich die Zugabe von Calcium unmittelbar vor Rückführen des Bluts in das Gefäßsystem des Patienten besonders anbietet. In diesem Bereich erfolgt keine Kontrolle des extrakorporalen Blutkreislaufs mehr über hierin ggf. vorhandene Lufteinschlüsse. Die vorliegende Erfindung schafft an dieser Stelle vorteilhaft Abhilfe, indem sie zur Vermeidung eines Luftembolierisikos eine Befüllung der Calciumleitung samt Überwachung des Ergebnisses des Primens bereits vor Behandlungsbeginn vorschlägt.

Ein weiterer erfindungsgemäßer Vorteil besteht darin, dass zum Befüllen der Calciumleitung für den Fall, dass bei der Vorbereitung des extrakorporalen Blutkreislaufs nicht bekannt ist, ob überhaupt Calciumlösung erforderlich sein wird (also bei initial nicht bekanntem Bedarf) nicht etwa die kostspielige Calciumlösung des Calciumlösungsbehälters verwendet wird, etwa mittels anterograder Befüllung, wie dies im Zusammenhang mit dem Anhängen von Infusionen bekannt ist. Vielmehr erfolgt das Primen mittels kostengünstiger Priminglösung.

Das erfindungsgemäße Primen erfolgt ferner vorteilhaft automatisiert. Ein manuelles Anhängen der Calciumleitung an beispielsweise eine Quelle für eine Priminglösung, ein anschließendes Lösen der Calciumleitung von der Quelle für eine Priminglösung, und ein Verbinden der Calciumleitung mit dem Blutkreislauf kann vorteilhaft entfallen.

Durch das Nichteinlegen oder das Lösen des Pumpschlauchabschnittes der Calciumleitung aus einer Calciumpumpe kann ein ungestörter Durchfluss durch den Pumpschlauchabschnitt hindurch vorteilhaft gewährleistet werden. Eine ggf. okkludierende Wirkung der Calciumpumpe kann umgangen werden, was ein Primen überhaupt erst ermöglichen oder zu einem gründlicheren Primen führen kann.

Durch das Nichteinlegen oder das Lösen kann das Befüllen der Calciumleitung ggf. vorteilhaft allein mittels der Schwerkraft oder mittels des Prinzips der kommunizierenden Gefäße stattfinden.

Beim Einlegen des Pumpschlauchabschnittes in die Calciumpumpe sinkt der Flüssigkeitsspiegel aufgrund des zusätzlich zugegebenen vorbestimmten Volumens an Priminglösung vorteilhaft allenfalls bis auf oder knapp oberhalb des vorbestimmten Detektionsspiegels; ein Spiegel bleibt daher für den Anwender dauerhaft erkennbar in der Calcium-Tropfkammer stehen.

Die vorliegende Erfindung wird im Folgenden anhand der beigefügten Zeichnung, in welcher identische Bezugszeichen gleiche oder ähnliche Bauteile bezeichnen, exemplarisch erläutert. In den zum Teil stark vereinfachten Figuren gilt:
- **Fig. 1**: zeigt schematisch vereinfacht eine Anordnung zum Durchführen einer Ausführungsform des erfindungsgemäßen Verfahrens in einem ersten Verfahrensschritt hiervon;
- **Fig. 2**: zeigt die Anordnung der Fig. 1 in einem sich anschließenden zweiten Verfahrensschritt; und
- **Fig. 3**: zeigt die Anordnung der Fig. 2 in einem sich anschließenden dritten Verfahrensschritt.

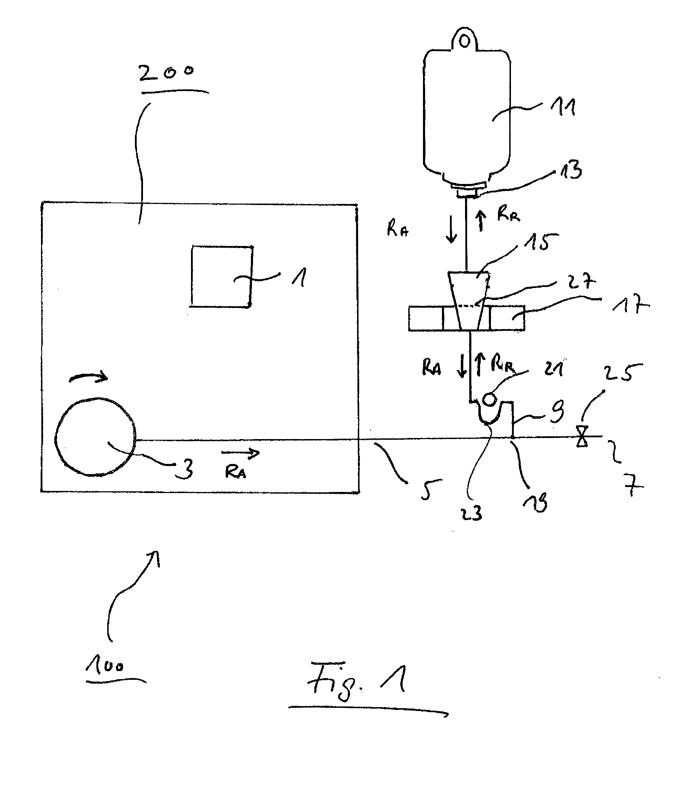

**Fig. 1** zeigt schematisch vereinfacht einen Blutkreislauf 100, welcher in einen Abschnitt einer Plasma- oder Blutbehandlungsvorrichtung 200 eingelegt ist, mittels welchem das erfindungsgemäße Verfahren stattfindet.

Der Abschnitt der Plasma- oder Blutbehandlungsvorrichtung 200 umfasst in der hier dargestellten Ausführungsform zusätzlich zu einer erfindungsgemäßen Steuereinrichtung 1 eine Blutpumpe 3, mittels welcher der Blutkreislauf 100 zu seinem Primen mit Priminglösung befüllt wird.

Der in Fig. 1 nur in Teilen hiervon dargestellte Blutkreislauf 100 umfasst eine Rückgabeleitung 5 (auch als venöse Leitung bezeichnet) mit einem Rückgabeanschluss 7 (auch als venöser Patientenanschluss bezeichnet) zur Rückgabe von Blut an den nicht dargestellten Patienten während der Blutbehandlung. In Fluidverbindung mit der Rückgabeleitung 5 steht eine Calciumleitung 9 als Beispiel einer Leitung zum Zugeben einer medizinischen Lösung, welche aus dem Calciumlösungsbehälter 11 gespeist wird oder werden kann, mit welchem sie mittels eines Calciumlösungsanschlusses 13 verbunden ist oder bei Bedarf verbunden wird. Der Calciumlösungsanschluss 13 kann als ein Spike ausgestaltet sein oder einen solchen aufweisen. Der Calciumlösungsanschluss 13 kann als eine Entlüftungseinrichtung ausgestaltet sein oder eine solche aufweisen.

Die Calciumleitung 9 weist eine Calcium-Tropfkammer 15 auf und ist, insbesondere im Bereich der Calcium-Tropfkammer 15, in eine Calcium-Tropf- und Spiegeldetektoreinrichtung 17 der Plasma- oder Blutbehandlungsvorrichtung 200 eingelegt oder hieran angeordnet. Die Calciumleitung 9 ist mittels eines Verbindungsabschnitts 19 an einem Verbindungspunkt in unbehinderter Fluidverbindung mit der Rückgabeleitung 5 verbunden.

Ein weiterer Abschnitt der Calciumleitung 9, welcher in oder an einer Calciumpumpe 21 der Plasma- oder BlutBehandlungsvorrichtung 200 angeordnet ist, wird als Pumpschlauchabschnitt 23 bezeichnet. Der Pumpschlauchabschnitt 23 ist in Fig. 1 nicht in die Calciumpumpe 21 eingelegt, er hängt vielmehr frei oder lose durch. Sein Lumen ist in diesem Zustand nicht durch die Calciumpumpe 21 verengt.

Die Calciumlösung, welche im Calciumlösungsbehälter 11 vorgehalten wird, wird nur im Bedarfsfall eingesetzt.

Beim Primen des Blutkreislaufs 100 wird die Priminglösung mittels der Blutpumpe 3 der Blut- oder Plasmabehandlungsvorrichtung 100 entlang der Rückgabeleitung 5 anterograd, also in der Richtung der Pfeile R_{A}, durch die Rückgabeleitung 5 hindurch gefördert. Am Verbindungsabschnitt 19 gelangt sie retrograd in die Calciumleitung 9 hinein und strömt in Richtung auf den Calciumlösungsbehälter 11 zu. Die Calciumleitung 9 wird somit retrograd in der Richtung der Pfeile R_{R} befüllt.

Die Befüllung der Calciumleitung 9 erfolgt erfindungsgemäß in retrograder Richtung, wie oben beschrieben und entlang der Pfeile R_{R} in Fig. 1. Die entgegengesetzte Richtung, als anterograde Richtung bezeichnet, ist in Fig. 1 durch die Pfeile R_{A} bezeichnet. "Anterograd" bezeichnet im Gegensatz hierzu somit die Strömungsrichtung der Calciumlösung ausgehend vom Calciumlösungsbehälter 11 zum Verbindungsabschnitt 19.

Das Primen oder Befüllen der Calciumleitung 9 erfolgt somit während des Primens des übrigen Blutkreislaufs 100 oder im selben Arbeitsschritt. Es erfolgt mittels der Blut- oder Plasmabehandlungsvorrichtung 100, insbesondere vor dem Beenden des Primingverfahrens des Blutkreislaufs 100. Das Primen erfolgt mittels der Priminglösung, mit welcher auch der Blutkreislauf 100 geprimt wurde, und es erfolgt in retrograder Richtung, was die Calciumleitung 9 betrifft.

Beim retrograden Befüllen oder Primen der Calciumleitung 9 ist es möglich, dass die Priminglösung innerhalb der Calciumleitung 9 allein durch die Schwerkraft oder durch das Prinzip der kommunizierenden Röhren oder Gefäße voranströmt. Alternativ oder unterstützend hierzu kann die Priminglösung mittels einer Förderpumpe - wie beispielsweise der Blutpumpe 3 oder einer rückwärts fördernden Calciumpumpe 21-gefördert werden.

Der Pumpschlauchabschnitt 23 der Calciumleitung 9 ist in einigen erfindungsgemäßen Ausführungsformen während oder zu Beginn des Primens, welches beispielsweise mittels 0,9%iger NaCl-Lösung erfolgt, nicht mit der Calciumpumpe 21 verbunden oder in diese eingelegt, jedenfalls nicht bevor das Primen auch der Calciumleitung 9 zunächst abgeschlossen, unterbrochen oder modifiziert wird. Auf diese Weise wird ein ungestörter Flüssigkeitsdurchfluss durch die Calciumleitung 9 gewährleistet.

Dabei kann der Pumpschlauchabschnitt 9 mittels eines Clips an der Calciumpumpe 21, insbesondere an einer Haltevorrichtung der Calciumpumpe 21, gehalten sein oder werden, wobei oder während der Pumpschlauchabschnitt 9 nicht in die Calciumpumpe 21 eingelegt ist.

Für das vorstehend beschriebene Vorgehen zum Primen der Calciumleitung 9 wird das Entweichen von Luft aus der Calciumleitung 9 ermöglicht, beispielsweise über den Calciumlösungsanschluss 13, oder mittels eines hier nicht gezeigten Luftabscheiders, wie er aus dem Stand der Technik bekannt ist, und/oder durch Offenlassen der Verbindung der Calciumleitung 9 zum Calciumlösungsbehälter 11.

Nur im Bedarfsfall, etwa bei Auftreten einer Hypokalzämie des Patienten, wird dann der Calciumlösungsbehälter 11 durch den Calciumlösungsanschluss 13 an die Calciumleitung 9 angeschlossen. Der Calciumlösungsbehälter 11 wird nur im Bedarfsfall geöffnet oder angebrochen. Sollte eine Calcium-Substitution nicht erforderlich sein, so bleibt der Calciumlösungsbehälter unberührt und kann in einer später erfolgenden Behandlungssitzung verwendet werden.

Eine Klemmeinrichtung 25 kann während des Befüllens der Calciumleitung 9 geschlossen sein. Somit kann das Erreichen eines vorbestimmten Detektionsspiegels 27 (gestrichelt dargestellt) in der Calcium-Tropfkammer 15 beschleunigt werden.

Das Befüllen der Calciumleitung 9 wird automatisch oder manuell gestoppt, wenn der vorbestimmte Detektionsspiegel 27 erreicht ist.

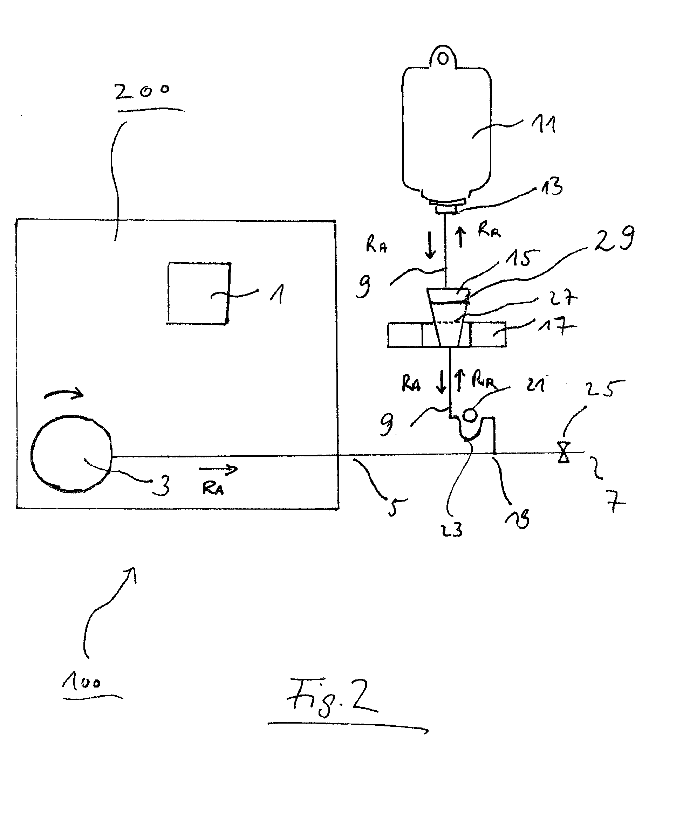

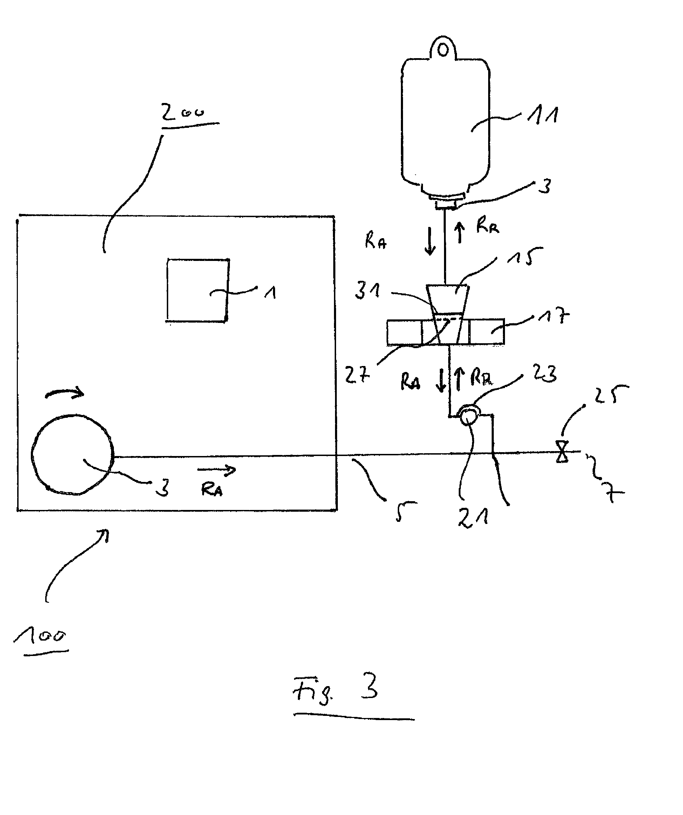

Zum Sicherstellen eines gewünschten After-Priming-Spiegels 31 (siehe Fig. 3) wird die Calciumleitung 9 um ein vorbestimmtes Flüssigkeitsvolumen befüllt. In der Calcium-Tropfkammer 15 wird damit ein Spiegel 29 erreicht, welcher oberhalb des Detektionsspiegels 27 liegt. Dies ist in **Fig. 2** gezeigt.

**Fig. 3** zeigt, dass der Pumpschlauchabschnitt 23 zwischenzeitlich - anders als in den in den Fig. 1 und 2 gezeigten ersten und zweiten Verfahrensschritten - in die Calciumpumpe 21 eingelegt ist, er hängt nicht mehr durch. Aufgrund des Einlegens ist der Spiegel 29 in der Calcium-Tropfkammer 15 (siehe Fig. 2) auf den After-Priming-Spiegel 31, einen stets für den Benutzer erkennbaren und damit leicht überprüfbaren Spiegel, abgesunken. Dieser After-Priming-Spiegel 31 liegt oberhalb des vorbestimmten Detektionsspiegels 27. Der Benutzer kann somit mit einem Blick prüfen, ob die Calciumleitung 9 ausreichend geprimt zur Verfügung steht.

### Bezugszeichenliste

- 1: Steuereinrichtung
- 3: Blutpumpe
- 5: Rückgabeleitung
- 7: Rückgabeanschluss zum Patienten
- 9: Calciumleitung
- 11: Calciumlösungsbehälter
- 13: Calciumlösungsanschluss
- 15: Calcium-Tropfkammer der Calcium-Tropf- und Spiegeldetektoreinrichtung 17
- 17: Calcium-Tropf- und Spiegeldetektoreinrichtung
- 19: Verbindungsabschnitt
- 21: Calciumpumpe
- 23: Pumpschlauchabschnitt
- 25: Klemmeinrichtung am Rückgabeanschluss 7
- 27: vorbestimmter Detektionsspiegel
- 29: Spiegel
- 31: After-Priming-Spiegel
- 100: extrakorporaler Blutkreislauf
- 200: Blut- oder Plasmabehandlungsvorrichtung
- R_{A}: anterograde Richtung
- R_{R}: retrograde Richtung

## Patentansprüche

1. Verfahren zum Primen einer von einer Rückgabeleitung (5) eines extrakorporalen Blutkreislaufs (100) abgehenden Leitung zum Zugeben einer medizinischen Lösung, wobei die Rückgabeleitung (5) eine venöse Patientenleitung ist, wobei die Leitung zum Zugeben einer medizinischen Lösung eine Calciumleitung (9) ist, durch den Schritt gekennzeichnet
- retrogrades Primen der Calciumleitung zum Zugeben von Calcium (9) über die Rückgabeleitung (5) des extrakorporalen Blutkreislaufs (100) mit einer Priminglösung,
wobei das retrograde Primen während des Primens des extrakorporalen Blutkreislaufs (100) mittels einer Blut- oder Plasmabehandlungsvorrichtung (200) erfolgt, welche als Apheresevorrichtung, Hämodialysevorrichtung, Hämofiltrationsvorrichtung oder Hämodiafiltrationsvorrichtung ausgestaltet ist.

2. Verfahren nach Anspruch 1, wobei die Calciumleitung (9), mit der Rückgabeleitung (5) des extrakorporalen Blutkreislaufs (100) einer Blut- oder Plasmabehandlungsvorrichtung (200) mittels eines Verbindungsabschnittes (19) lösbar oder unlösbar in Fluidkommunikation verbunden ist.

3. Verfahren nach einem der vorangegangenen Ansprüche, mit dem Schritt:
- Lösen eines Pumpschlauchabschnittes (23) der Calciumleitung (9) aus einer Calciumpumpe (21).

4. Verfahren nach einem der vorangegangenen Ansprüche, mit dem zusätzlichen Schritt:
- Unterbrechen, Modifizieren oder Beenden des retrograden Primens der Calciumleitung (9), nachdem ein vorbestimmter Detektionsspiegel (27) der Priminglösung in einer Calcium-Tropfkammer (15), welche in oder an der Calciumleitung (9) angeordnet ist, erreicht und/oder festgestellt ist.

5. Verfahren nach Anspruch 4, mit dem zusätzlichen Schritt:
- Unterbrechen, Modifizieren oder Beenden des retrograden Primens der Calciumleitung (9), nachdem oder wenn ein vorbestimmter Detektionsspiegel (27) der Priminglösung in der Calcium-Tropfkammer (15) erreicht oder festgestellt ist, wobei der Detektionsspiegel (25) von einer Calcium-Tropf- und Spiegeldetektoreinrichtung (17) erkannt wird, welche an der Calcium-Tropfkammer (15) angeordnet ist.

6. Verfahren nach einem der vorangegangenen Ansprüche 4 bis 5, mit dem zusätzlichen Schritt:
- Weiteres Befüllen der Calcium-Tropfkammer (15) mit der Priminglösung um ein vorbestimmtes Volumen an Priminglösung, nachdem der vorbestimmte Detektionsspiegel (27) erreicht oder durch die Calcium-Tropf- und Spiegeldetektoreinrichtung (17) festgestellt ist.

7. Verfahren nach einem der vorangegangenen Ansprüche, mit dem zusätzlichen Schritt:
- Einlegen des Pumpschlauchabschnittes (23) in die Calciumpumpe (21).

8. Verfahren nach einem der vorangegangenen Ansprüche, mit dem zusätzlichen Schritt:
- Verändern des Durchflusses oder der Strömungsrate der Priminglösung durch die Rückgabeleitung (5) mittels einer Klemmeinrichtung (25), welche an der Rückgabeleitung (5) stromabwärts des Verbindungsabschnittes (19) oder stromabwärts eines Verbindungspunkts zur Calciumleitung (9) angeordnet ist.

9. Steuereinrichtung (1) konfiguriert zum Steuern oder Regeln des Verfahrens nach einem der vorangegangenen Ansprüche.

10. Blut- oder Plasmabehandlungsvorrichtung (200) mit wenigstens einer Steuereinrichtung (1) gemäß Anspruch 9.

11. Blut- oder Plasmabehandlungsvorrichtung (200) nach Anspruch 10 mit wenigstens einer Calcium-Tropf- und Spiegeldetektoreinrichtung (17) zum Detektieren eines Flüssigkeitsspiegels in wenigstens einer Calcium-Tropfkammer (15) einer Calciumleitung (9), eines mit der Blut- oder Plasmabehandlungsvorrichtung (200) verbundenen extrakorporalen Blutkreislaufs (100).

12. Digitales Speichermedium, insbesondere in Form einer Diskette, CD oder DVD oder EPROM, mit elektronisch auslesbaren Steuersignalen, konfiguriert, um derart mit einem programmierbaren Computersystem zusammenzuwirken, dass die maschinellen Schritte eines erfindungsgemäßen Verfahrens nach einem der Ansprüche 1 bis 8 veranlasst werden.

13. Computerprogramm-Produkt mit einem auf einem maschinenlesbaren Träger gespeicherten Programmcode zur Veranlassung der maschinellen Schritte des erfindungsgemäßen Verfahrens gemäß einem der Ansprüche 1 bis 8, wenn das Computerprogramm-Produkt auf einem Rechner abläuft.

## Claims

1. A method for priming a line which branches off a return line (5) of an extracorporeal blood circuit (100) for adding a medical solution, wherein the return line (5) is a venous patient line, wherein the line for adding the medical solution is a calcium line (9), **characterized by** the step
- retrograde priming of the calcium line (9), via the return line (5) of the extracorporeal blood circuit (100) with a priming solution,
wherein the retrograde priming takes place while the extracorporeal blood circuit (100) is primed by means of a blood or plasma treatment apparatus (200) which is designed as an apheresis apparatus, a hemodialysis apparatus, a hemofiltration apparatus or a hemodiafiltration apparatus.

2. The method according to claim 1, wherein the calcium line (9) is connected in fluid communication with the return line (5) of the extracorporeal blood circuit (100) of a blood or plasma treatment apparatus (200) by means of a connection section (19) in a detachable or undetachable way.

3. The method according to one of the preceding claims, with the step:
- detaching a pump tube section (23) of the calcium line (9) from a calcium pump (21).

4. The method according to one of the preceding claims, with the additional step:
- disrupting, modifying or stopping the retrograde priming of the calcium line (9), after a predetermined detection level (27) of the priming solution is or was reached and/or detected in a calcium drip chamber (15), which is arranged in or at the calcium line (9).

5. The method according to claim 4, with the additional step:
- disrupting, modifying or stopping the retrograde priming of the calcium line (9), after or when a predetermined detection level (27) of the priming solution is or was reached and/or detected in the calcium drip chamber (15), wherein the detection level (25) is detected by a calcium drip and level detector device (17), which is arranged at the calcium drip chamber (15).

6. The method according to one of the preceding claims 4 to 5, with the additional step:
- further filling of the calcium drip chamber (15) with the priming solution by a predetermined volume of priming solution, after the predetermined detection level (27) is or was reached or detected by the calcium drip and level detector device (17).

7. The method according to one of the preceding claims, with the additional step:
- inserting the pump tube section (23) into the calcium pump (21).

8. The method according to one of the preceding claims, with the additional step:
- changing the flow or the flow rate of the priming solution through the return line (5) by means of a clamping device (25), which is arranged at the return line (5) downstream from the connection section (19) or downstream from a connection spot to the calcium line (9).

9. A control device (1) configured for controlling or regulating the method according to one of the preceding claims.

10. A blood or plasma treatment apparatus (200) with at least one control device (1) according to claim 9.

11. The blood or plasma treatment apparatus (200) according to claim 10 with at least one calcium drip and level detector device (17) for detecting a level of liquid in at least one calcium drip chamber (15) of a calcium line (9), of an extracorporeal blood circuit (100) which is connected with the blood or plasma treatment apparatus (200).

12. A digital storage medium, in particular in the form of a disk, CD or DVD or EPROM, with electronically readable control signals, configured for interacting with a programmable computer system such that the machine steps of a method according to the invention according to one of the claims 1 to 8 are prompted.

13. A computer program product with a program code saved on a machine-readable medium for prompting the machine steps of a method according to the invention according to one of the claims 1 to 8 when the computer program product runs on a computer.

## Revendications

1. Procédé pour l'amorçage d'une ligne d'ajout d'une solution médicale, ladite ligne dérivant d'une ligne de retour d'un circuit sanguin extracorporel (100), la ligne de retour (5) étant une ligne veineuse d'un patient et la ligne d'ajout de la solution médicale une ligne de calcium (9), **caractérisé par** l'étape
- amorcement rétrograde de la ligne de calcium (9) par la ligne de retour (5) du circuit sanguin extracorporel (100) au moyen d'une solution d'amorçage,
où l'amorçage rétrograde a lieu lors de l'amorçage du circuit sanguin extracorporel (100) au moyen d'un appareil de traitement du sang ou du plasma lequel est configuré comme appareil d'aphérèse, d'hémodialyse, d'hémofiltration ou d'hémodiafiltration.

2. Procédé selon la selon la revendication 1, dans lequel la ligne de calcium (9) est connectée en communication fluidique avec la ligne de retour (5) du circuit sanguin extracorporel (100) de l'appareil de traitement du sang ou du plasma (200) de manière détachable ou non détachable au moyen d'un segment de raccordement (19).

3. Procédé selon l'une des revendications précédentes, comprenant l'étape :
- détacher une section de tube de pompe (23) de la ligne de calcium (9) d'une pompe de calcium (21).

4. Procédé selon l'une des revendications précédentes, comprenant l'étape :
- interrompre, modifier ou arrêter l'amorçage rétrograde de la ligne de calcium (9) après qu'un niveau de détection prédéterminé (27) de la solution d'amorçage est ou a été atteint et / ou détecté dans une chambre de goutte à goutte de calcium (15) disposée dans ou à la ligne de calcium (9).

5. Procédé selon la revendication 4, comprenant l'étape supplémentaire :
- interrompre, modifier ou arrêter l'amorçage rétrograde de la ligne de calcium (9), après que ou lorsqu'un niveau de détection prédéterminé (27) de la solution d'amorçage est ou a été atteint et / ou détecté dans la chambre de goutte à goutte de calcium (15), où le niveau de détection (25) est détecté par un détecteur de goutte à goutte de calcium et de niveau (17) disposé à la chambre de goutte à goutte de calcium (15).

6. Procédé selon l'une des revendications 4 ou 5, comprenant l'étape supplémentaire :
- remplissage supplémentaire de la chambre de goutte à goutte de calcium (15) avec un volume prédéterminé de la solution d'amorçage, après que le niveau de détection prédéterminé (27) est ou a été atteint et / ou détecté par le détecteur de goutte à goutte et de niveau (17).

7. Procédé selon l'une des revendications précédentes, comprenant l'étape supplémentaire :
- insérer la section de tube de pompe (23) dans la pompe de calcium (21).

8. Procédé selon l'une des revendications précédentes, comprenant l'étape supplémentaire :
- modifier le débit ou le taux d'écoulement de la solution d'amorçage dans la ligne de retour (5) au moyen d'un dispositif de serrage (25) disposé sur la ligne de retour (5) en aval du segment de raccordement (19) ou en aval d'un point de raccordement à la ligne de calcium (9).

9. Dispositif de régulation (1) configuré pour contrôler ou réguler un procédé selon l'une des revendications précédentes.

10. Appareil de traitement du sang ou du plasma (200) comprenant au moins un dispositif de régulation (1) selon la revendication 9.

11. Appareil de traitement du sang ou du plasma (200) selon la revendication 10 comprenant au moins un détecteur de goutte à goutte de calcium et de niveau (17) pour détecter un niveau de liquide dans au moins une chambre de goutte à goutte de calcium (15) d'une ligne de calcium (9) d'un circuit sanguin extracorporel relié à l'appareil de traitement de sang ou de plasma (200).

12. Un support de stockage digital, en particulier sous la forme d'un disque, CD, DVD ou EPROM, comprenant des signaux de contrôle électroniques lisibles, configurés pour interagir avec un système informatique programmable de façon les étapes à ce que non transitoire par ordinateur avec un programme exécutable stocké sur celui-ci, dans lequel le programme ordonne à un système d'ordinateur programmable de façon à exécuter les étapes machinelles du procédé selon l'une des revendications 1 à 8.

13. Programme d'ordinateur en tant que produit comprenant un code de programmation stocké sur un support lisible par une machine pour exécuter les étapes machinelles du procédé selon l'une des revendications 1 à 8 lorsque le programme d'ordinateur est exécuté par un ordinateur.
